# EUROPEAN PATENT APPLICATION

(11) **EP 2 389 010 A1**
(43) Date of publication of application: **23.11.2011**
(21) Application number: 11380032.0
(22) Date of filing: 02.03.2011
(51) Int. Cl.: H04R 1/12, B65D 81/24, A61B 7/02

(54) **Single use protector for stethoscope bells and membranes with a tab for easy removal**

(30) Priority: 03.03.2010 ES 201000298
(71) Applicant: Castillo Vaquera, Jose, 06011 Badajoz (ES)
(72) Inventor: Castillo Vaquera, Jose, 06011 Badajoz (ES); Zayas Hinojosa, Fernando, . (ES)

(57) **Abstract**

The single use protector (1) consists of a bag made up from a circular piece of non woven fabric showing in a small part of its perimeter a tab (2), used to easily remove the protector when installed in the stethoscope. The protector fits to the shape of the bell of the stethoscope by sewing a pre-stretched elastic band (3) around the whole edge of the circular piece of fabric The removal tab (2) makes easy to remove the protector without having to touch the surface that has been in contact with the skin of the patient.

The protector (1) may be built in different materials and adaptable to different types of stethoscopes, brands, sizes existing in the market. Also, the protector (1) may be made up to cover the membrane and the bell, or the whole terminal portion of the stethoscope, including part of the tube of the stethoscope.

## Description

### TECHNICAL SECTOR FOR APPLICATION

Single use clinic materials.

### PRECEDENTS OF INVENTION

In clinical practising the auscultation of body noises is performed by using stethoscopes with a membrane and an amplifier bell.

These membranes and bells are directly applied over several areas of the patient's skin, depending of the type of corporal noise trying to be detected.

Also, these stethoscopes are sequentially used on different patients, generally without being disinfected between uses, thus no complying the minimal hygienic requisites required by a good quality medical assistance.

Many scientific reports set clear that using the same objects in different patients is a main driver in diseases transmission, especially when those objects come in contact with biological remains of a patient. These remains may contain pathogens that so find an ideal transmission form among different persons.

The protector proposed by this invention avoids, at low cost, the inconveniences previously described, due to the barrier imposed between the membrane and the stethoscope and the patient, preventing the transmission of pathogens.

### DESCRIPTION

The present invention consists of a single use protector with a removal tab, made of non woven fabric that also can be covered with a waterproof polymer, adaptable to the shape of the bell used in the stethoscopes.

### Description of a preferred building process

A more detailed description of the invention follows, making reference to the included figures as an example of a configuration that is not completely exhaustive, although responses to a preferred way of construction.

The single use protector (1) consists of a bag made up from a circular piece of non woven fabric showing in a small part of its perimeter a tab (2), used to easily remove the protector when installed in the stethoscope. The protector fits to the shape of the bell of the stethoscope by sewing a pre-stretched elastic band (3) around the whole edge of the circular piece of fabric.

The elastic band (3) may be made up of one of the threads needed to machine stitch it to the protector.

The removal tab (2) makes easy to remove the protector without having to touch the surface that has been in contact with the skin of the patient.

This tab (2) may be reinforced by means of an edge stitching in order to avoid the risk of tearing the invention during the operation of removal it from the stethoscope. As an alternative, the stitching may cover the whole edge of the tab (2) or only in one of the sides.

The protector (1) may alternatively have no removal tab (2) or present more than one.

The elastic band (3) may alternatively be stitched only in the edge of the protector (1) without including the part where the tab (2) exists.

The protector (1) may be covered with a sheet of polymer to make the invention water proof.

Also, the protector (1) may be made up of non woven fabric of different textures and densities, or in other different types of materials of similar characteristics that those of the non woven fabric.

The protector (1) may be built adaptable to different types of stethoscopes, brands, sizes existing in the market. Also, the protector (1) may be made up to cover the membrane and the bell, or the whole terminal portion of the stethoscope, including part of the tube of the stethoscope.

The protector may be made of different colours and may be identified with letters, figures, logos, etc.

### DESCRIPTION OF FIGURES

Figure 1 presents the invention and it shows the shape of the protector (1) and the removal tab (2).
Figure 2 shows the form of the template to cut the sheet of material used to build the protector (1). It has the shape of a disk with a rectangular projection to form the removal tab (2).

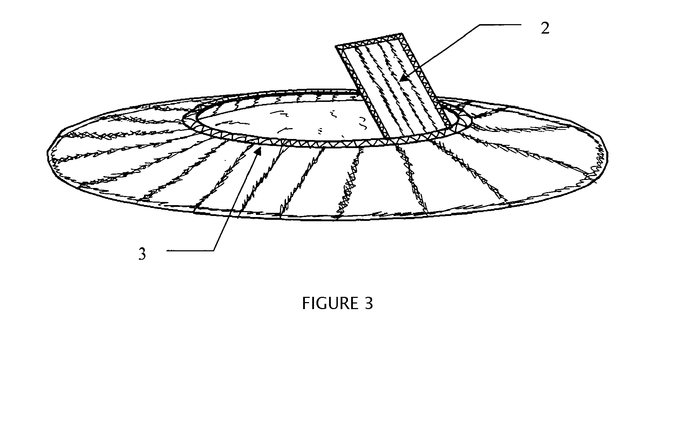

Figure 3 presents the invention once adapted to the stethoscope, although in the figure it has not been represented the whole stethoscope in order to make clearer the shape of the invention. Also the removal tab (2) and the elastic band (3) can be observed in the figure.

### MODE OF COMPLETION

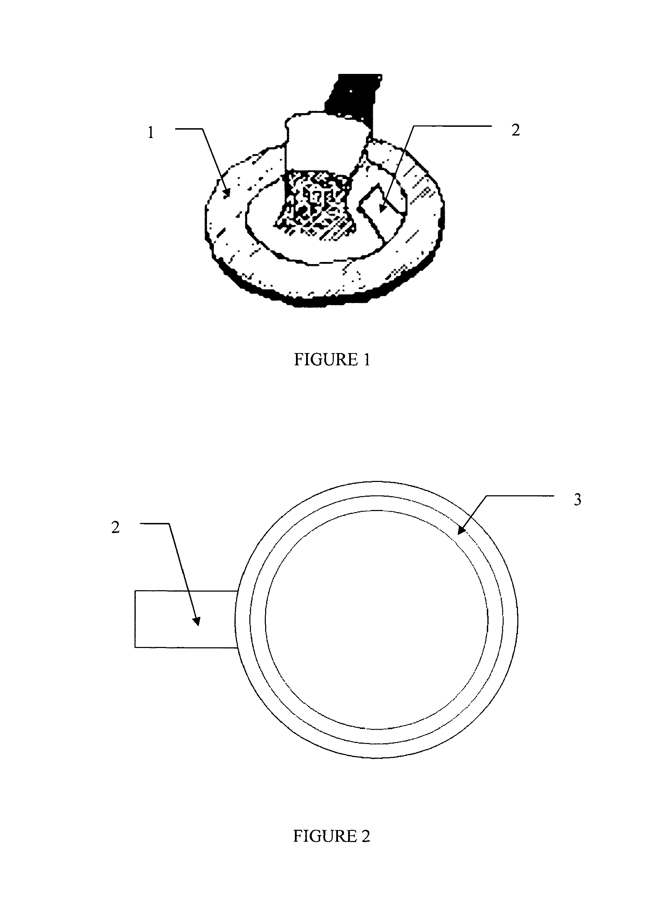

The invention, as can be seen in Figure 1, is conformed from a piece of fabric cut with the form of the template as it is seen in Figure 2. After this, an elastic band (3) is stitched pre-stretched to the circular edge of the piece of fabric. The removal tab (2) may have the elastic band stitched in three straight edges and alternatively may only have a straight elastic band stitched along the centreline of the tab or in one of the sides.

## Claims

1. Single use protector (1) for stethoscope bells and membranes, with a tab (2) for easy removal, **characterized by** being made up from a circular piece of non woven fabric, conformed by stitching in its edge a pre-stretched elastic band (3).

2. Single use protector (1) for stethoscope bells and membranes, with a tab (2) for easy removal, according claim 1, in which the elastic band (3) may be one of the threads needed to perform the stitching in the edge by mechanical means.

3. Single use protector (1) for stethoscope bells and membranes, with a tab (2) for easy removal, according claim 1, in which the tab (2) may be reinforced with an edge stitching to avoid tearing of the fabric during the process of removal the protector (1) from the stethoscope.

4. Single use protector (1) for stethoscope bells and membranes, with a tab (2) for easy removal, according claims 1 and 3, in which the tab (2), as an alternative to the edge stitching, may be reinforced only by a straight elastic band stitched along the centreline of the tab or in one sides.

5. Single use protector (1) for stethoscope bells and membranes, with a tab (2) for easy removal, according claims 1, 3 and 4, in which there may have conformed more than one tab.

6. Single use protector (1) for stethoscope bells and membranes, with a tab (2) for easy removal, according claims 1 and 2, in which as an alternative the elastic band (3) may be stitched in the circular edge but not in the part of the edge where the tab (2) is attached, thus leaving a part of the protector (1) without elastic band.

7. Single use protector (1) for stethoscope bells and membranes, with a tab (2) for easy removal, according claims 1,2 ,3, 4, 5, and 6, in which as an alternative the elastic band (3) pre-stretched may be stitched along the whole perimeter of the protector, including the tab (2).

8. Single use protector (1) for stethoscope bells and membranes, with a tab (2) for easy removal, according claim 1, in which the protector may be built from non woven fabric covered with a sheet of polymer to make the invention water proof.

9. Single use protector (1) for stethoscope bells and membranes, with a tab (2) for easy removal, according claim 1, in which the protector may be made up of non woven fabric of different colours, textures and densities, or in other different types of fabrics with similar characteristics that those of the non woven fabric

10. Single use protector (1) for stethoscope bells and membranes, with a tab (2) for easy removal, according claim 1, may be built adaptable to stethoscopes of two bells, different types of stethoscopes, brands, sizes existing in the market.

11. Single use protector (1) for stethoscope bells and membranes, with a tab (2) for easy removal, according claim 1, in which the preformed protector may cover only the bell and membrane, or the whole terminal portion of the stethoscope, including the final part of the rubber tube.

12. Single use protector (1) for stethoscope bells and membranes, with a tab (2) for easy removal, according claim 1, in which the protector may be built in other materials different to the non woven fabric.

13. Single use protector (1) for stethoscope bells and membranes, with a tab (2) for easy removal, according claim 1, in which the protector may be built without the removal tab (2).
